(19) Europäisches Patentamt
European Patent Office
Office européen des brevets

(11) Publication number : **0 514 090 A1**

(12) # EUROPEAN PATENT APPLICATION

(21) Application number : **92304151.1**

(22) Date of filing : **08.05.92**

(51) Int. Cl.$^5$ : **C07H 19/01,** C07D 493/22, A01N 43/90, A61K 31/70, // (C07D493/22, 313:00, 311:00, 311:00, 307:00)

(30) Priority : **13.05.91 US 698874**

(43) Date of publication of application : **19.11.92 Bulletin 92/47**

(84) Designated Contracting States : **CH DE FR GB IT LI NL**

(71) Applicant : **MERCK & CO. INC.**
**126, East Lincoln Avenue P.O. Box 2000**
**Rahway New Jersey 07065-0900 (US)**

(72) Inventor : **Jones, Todd K.**
**99-B Hana Road**
**Edison, NJ 08817 (US)**
Inventor : **Mrozik, Helmut**
**159 Idlebrook Lane**
**Matawan, NJ 07747 (US)**

(74) Representative : **Thompson, John Dr. et al**
**Merck & Co., Inc. European Patent Department Terlings Park Eastwick Road**
**Harlow, Essex CM20 2QR (GB)**

(54) **Procedure for the inversion of the stereochemistry at C13 of avermectin aglycone compounds.**

(57) The natural stereochemistry at the 13-position of avermectin aglycones, normally α-oriented or below the plane of the molecule, is inverted or epimerized into the β-position. The procedure starts with the avermectin aglycone compounds where the 13α-hydroxy group is substituted with a leaving group. Treatment of the substituted compound with tetra alkyl ammonium nitrate displaces the leaving group, substitutes a nitrate ester or a nitrooxy group, for the hydroxy and inverts the 13-stereochemistry to β. Reduction of the nitrooxy group returns the 13-hydroxy group and retains the 13-β configuration. The intermediate 13-nitrooxy compounds are themselves potent anti-parasitic agents.

EP 0 514 090 A1

## BACKGROUND OF THE INVENTION

Avermectin compounds are well known antiparasitic agents useful in animal health, human health and agriculture. The avermectin compounds, and the related milbemycin compounds are prepared micro-biologically and are highly complex organic molecules. See Albers-Schoenberg et al, U.S. Patent 4,310,519 and Chabala et al, U.S. Patent 4,199,569. The natural stereochemistry of the 13-position substituent of the avermectins is a, with the oxygen atom below the plane of the ring.

Prior efforts at inverting the 13-position stereochemistry were complex, multi-step syntheses that resulted in poor yields and mixtures of isomers. See Mrozik et al, J. Med. Chem., 32 pg 375-381 (1989); In addition, efforts at preparing the 13-β compounds have not been successful when traditional epimerization procedures were attempted such as the well-known Mitsunobu inversion reaction. See Mitsunobu, Synthesis pg 1-28 (1981) and Hughes et al, J. Am. Chem. Soc., 110 pg 6487-6491 (1988). See also Cainelli et al, Tetrahedron Lett. 26 pg 3369-3372 (1985) and Cainelli et al, Tetrahedron 41 pg 1385-1892 (1985) for a general description of an inversion process involving nucleophilic displacement promoted by nitrate ions. Of all of the procedures attempted, the inversion of the 13-position stereochemistry is most readily accomplished as described below with the highest yields of any such processes.

## SUMMARY OF THE INVENTION

The instant invention is concerned with a procedure for the inversion of the 13-position stereochemistry of the compounds. Thus, it is an object of the instant invention to describe a process for the efficient preparation of avermectin aglycone compounds with reversed 13-position stereochemistry. A further object is to describe the specific reaction conditions which most efficiently produce the stereochemically reversed compounds. A still further object is to describe the intermediates used in the inversion process. Still further objects will become apparent from a reading of the following description.

## DESCRIPTION OF THE INVENTION

The process of inverting the natural α-stereochemistry of the avermectin 13-position hydroxy into the inverted or β-configuration, also sometimes referred to as 13-epi-aglycones, is outlined in the following reaction scheme.

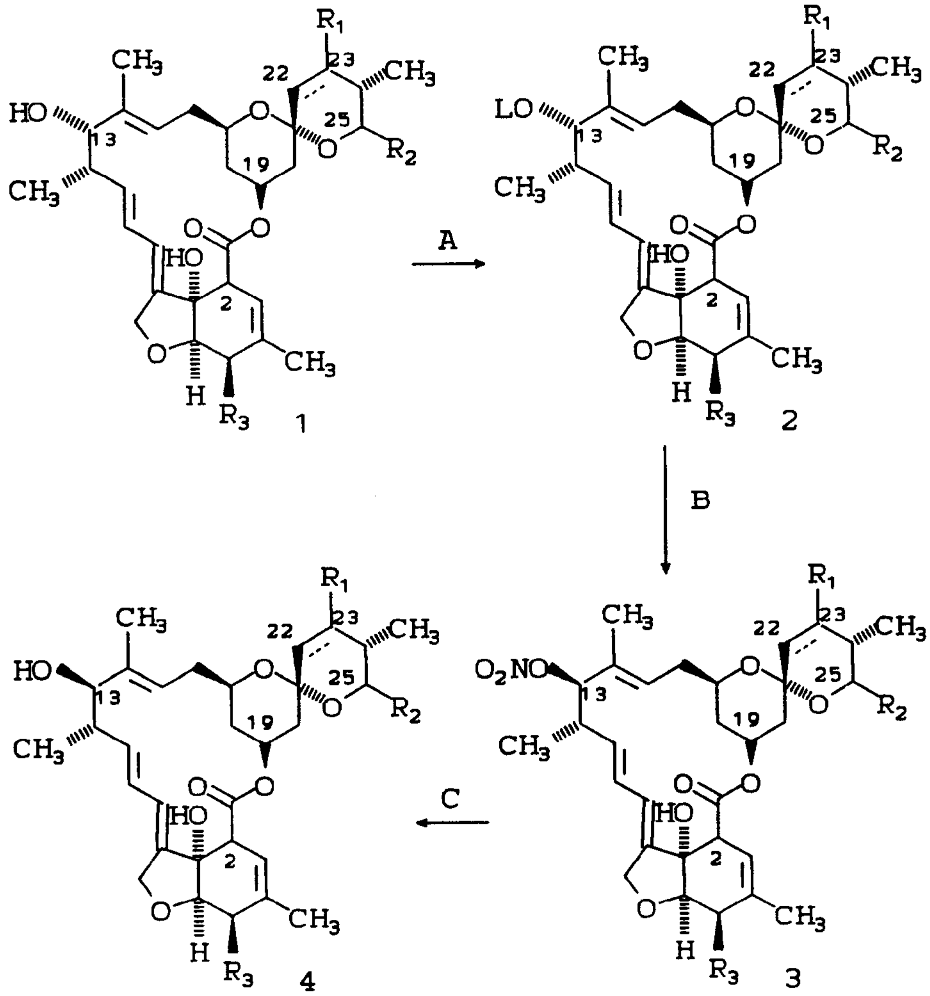


1 2 3 4

where L is a leaving group and the broken line at the 22,23-position indicates either a single or a double bond at the 22,23-position;

$R_1$ is present only when the broken line indicates a 22,23-single bond and is hydrogen, hydroxy, or oxo;

$R_2$ is loweralkyl, loweralkenyl, or cycloloweralkyl;

and

$R_3$ is hydroxy, loweralkoxy, oximino or oxo.

In the instant application, the following terms are intended to have the following definitions:

The term "loweralkyl" is intended to include those alkyl groups containing from 1 to 6 carbon atoms in either a straight or branched configuration. Examples of such alkyl groups are methyl, ethyl, n-propyl, isopropyl, n-butyl, sec-butyl, isobutyl, tert-butyl, n-pentyl, iso-pentyl, neo-pentyl, n-hexyl, iso-hexyl and the like.

The term "loweralkenyl" is intended to include those alkenyl groups of from 2 to 6 carbon atoms in either a straight or branched configuration. Examples of such alkenyl groups are ethenyl, 1-propenyl, 2-propenyl, 1-butenyl, 2-butenyl, 1-pentenyl, 4-methyl-2-penten-2-yl, 3-pentenyl, 1-hexenyl, 2-hexenyl and the like.

The term "cycloalkyl", is intended to include those cycloalkyl groups of from 3 to 8 carbon atoms. Examples of such cycloalkyl groups are cyclopropyl, cyclobutyl, cyclopentyl, cyclohexyl, cycloheptyl and cyclooctyl.

The term "loweralkoxy" is intended to include those alkoxy groups of from 1 to 6 carbon atoms in either a straight or branched configuration. Examples of such alkoxy groups are methoxy, ethoxy, propoxy, iso-propoxy, butoxy, tert-butoxy, pentoxy, sec-pentoxy, hexoxy and the like.

The instant invention also includes the intermediate nitrooxy compounds, 3 above, as novel compounds, both as intermediates in the preparation of 13-epi compounds and as active anthelmintic agents themselves. As active anthelmintic agents certain compounds are preferred over the others. Such preferred compounds are realized in the above structural formula when the broken line indicates a 22,23-single bond and $R_1$ is hy-

drogen, hydroxy or oxo;
$R_2$ is alkyl of from 3 to 5 carbon atoms or cycloalkyl of from 4 to 6 carbon atoms; and
$R_3$ is hydroxy, methoxy, oximino or oxo.

Further preferred compounds are realized when $R_1$ is hydrogen or oxo;
$R_2$ is alkyl of 3 to 4 carbon atoms or cycloalkyl of 5 to 6 carbon atoms; and
$R_3$ is hydroxy or oximino.

Still further preferred compounds are realized when $R_1$ is hydrogen;
$R_2$ is isopropyl, sec-butyl, cyclohexyl or cyclopentyl; and
$R_3$ is hydroxy.

The process for the inversion of the 13-position stereochemistry from α- to β- begins with the avermectin aglycone compound with a free 13-hydroxy group and any additional hydroxy group, such as those at the 5 and 23-positions, protected to prevent reactions at those positions. It is noted that the 7-position hydroxy group is generally non-reactive under the reaction conditions employed in the instant series of reactions and need not be protected.

Subsequent to any of the above described reactions the protecting group may be removed and the unprotected product isolated. The protecting group employed is ideally one which may be readily synthesized, will not be affected by the reactions at the other positions And may be readily removed without affecting any other functions of the molecule. One preferred type of protecting group for the avermectin type of molecule is the trisubstituted silyl group, preferably the trialkyl silyl group. One especially preferred example, is the t-butyl dimethylsilyl group. The reaction preparing the protected compounds is carried out by reacting the hydroxy compounds with the appropriately substituted silylhalide, preferably the silylchloride in an aprotic polar solvent such as dimethylformamide. Imidazole is added as a catalyst. The reaction is complete in from 1 to 24 hours and at from 0° to 25°C. For the 5-position hydroxy group the reaction is complete in from 1/2 to 3 hours at from 0°C to room temperature. This reaction is selective to the 5-position under the conditions above described and very little, if any, silylation is observed at other hydroxy substituted positions. If it is desired to protect the 23-hydroxy group a 5,23-di(phenoxyacetyl) derivative or a 23-silyl ether can be prepared. Basic hydrolysis will leave the highly hindered 23-O-substituent but hydrolyze the 5- -O-phenoxy acetyl groups. The 5-position is then protected as described above, selectively with a t-butyldimethylsilyl group.

The silyl group or groups are removed by stirring the silyl compound in methanol catalyzed by an acid preferably a sulfonic acid hydrate such as methanolic 1.0% p-toluene sulfonic acid monohydrate. The reaction is complete in about 1 to 12 hours at from 0° to 50°C. Alternatively the silyl group or groups may be removed by treatment of the silyl compound with anhydrous pyridine-hydrogen fluoride in tetrahydrofuran. The reaction is complete in from 3 to 24 hours at from 0° to 25°C.

The first step in the inversion of the 13-position stereochemistry involves the substitution of a leaving group (L) on the suitably protected 13-hydroxy compound, Step A. The nature of the leaving group is not significant so long as it can be readily substituted on Compound 1 at the 13-hydroxy position and is readily displaced in Step B of the instant process. Preferred leaving groups are acyl groups such as sulfonic acid derivatives with the p-toluene sulfonic acid derivative (tosylate) most preferred. The sulfonic acid derivative is readily prepared from the corresponding anhydride or chloride although the use of the anhydride is preferred since that will avoid the preparation of unwanted chloride side-products which lowers yields. The sulfonic acid derivatives are conveniently prepared in a non-polar solvent such as a chloronated hydrocarbon preferably chloroform or methylene chloride at from 0°C to the reflux temperature of the reaction mixture, although room temperature is preferred. The reaction is generally complete in from 1 to 24 hours and the product, Compound 2, is isolated using techniques known to those skilled in the art.

Generally, it has been found that the presence of a base, preferably an organic base such as alkyl substituted amine or pyridine, will facilitate the substitution of the sulfonic acid at the 13-position hydroxy.

The second step, Step B, of the reaction sequence involves the displacement of the leaving group by a nitrooxy group and the simultaneous inversion of the 13-position stereochemistry. The displacement of the leaving group is most conveniently carried out by reacting Compound 2 with a tetra loweralkyl ammonium nitrate, preferably a tetrabutyl ammonium nitrate. The reaction is carried out in an inert solvent, preferably a non-polar solvent such as a chlorinated hydrocarbon such as chloroform or methylene chloride or a hydrocarbon such as benzene, toluene and the like. Toluene is preferred. The reaction is run at from 25°C to 100°C preferably about 40°C and is complete in from 10 minutes to 48 hours. The 13-epi nitro substituted hydroxy compound (Compound 3) is isolated using techniques known to those skilled in the art.

The nitrooxy group is hydrolyzed in Step C without again reversing the 13-position stereochemistry by reduction with zinc in the presence of an acid. The zinc is present in an form such as powder, granular, lump, etc., although the reaction is most efficient if powdered zinc is used. The reaction is carried out in any solvent in which Compound 3 is soluble or slightly soluble such as the hydrocarbon solvents listed above for Step B

and polar solvents such as lower alkanols such as methanol, ethanol and the like, tetrahydrofuran, ether, and other solvents.

The acid is preferably an organic acid such as acetic acid and is present in a large molar excess over the starting material, preferably at from 5 to 50% of the volume of the reaction mixture, and most preferably about 15% of the volume of the reaction mixture. The reaction is exothermic and initially is carried out with cooling in order to moderate the temperature of the reaction, however, temperatures of from 0°C to the boiling-point of the reaction mixture may be employed. The reaction is generally complete in from 5 minutes to 10 hours and the product is isolated using techniques known to those skilled in the art. Following the isolation of the 13-epi hydroxy compound 4, any protecting groups may be removed as described above in order to obtain the desired final product.

The preparation of derivatives of avermectin aglycone compounds which are used as the starting materials for the instant process are well described in the scientific and patent literature. See, for example U.S. Patent 4,310,819 to Albers-Schoenberg et al, for the avermectin natural products; U.S. Patent 4,199,569 to Chabala et al, for the 22,23-dihydroavermectin derivates; U.S. Patent 4,906,619 to Eskola et al, for the preparation of various alkylated avermectins; U.S. Patent 4,200,981 to Fisher et al, for the preparation of various 5-alkylated compounds; U.S. Patent 4,995,837 to Mrozik for a discussion of various procedures for the protection of avermectin compounds; European Patent application 214,731 for the preparation of various 25-alkyl, alkenyl and cycloalkyl derivatives; see also Chen et al, Abstr. Pap. Am. Chem. Soc., (186 Mtg MBTD 28 (1983)) for additional 25-substituted compounds; and Fisher et al, in Macrolide, Antibiotics, Omura, S (ed), Academic Press, New York and Davies et al, in Nat. Prod. Rep. 3 pg 87-121 (1986) reviews the literature of avermectin and milbemycin compounds for the preparation of additional compounds.

The instant compounds are potent endo- and ecto-antiparasitic agents against parasites particularly helminths, ectoparasites, insects, and acarides, infecting man, animals and plants, thus having utility in human and animal health, agriculture and pest control in household and commercial areas.

The disease or group of diseases described generally as helminthiasis is due to infection of an animal host with parasitic worms known as helminths. Helminthiasis is a prevalent and serious economic problem in domesticated animals such as swine, sheep, horses, cattle, goats, dogs, cats, fish, buffalo, camels, llamas, reindeer, laboratory animals, fur-bearing animals, zoo animals and exotic species and poultry. Among the helminths, the group of worms described as nematodes causes widespread and often times serious infection in various species of animals. The most common genera of nematodes infecting the animals referred to above are Haemonchus, Trichostrongylus, Ostertagia, Nematodirus, Cooperia, Ascaris, Bunostomum, Oesophagostomum, Chabertia, Trichuris, Strongylus, Trichonema, Dictyocaulus, Capillaria, Habronema, Druschia, Heterakis, Toxocara, Ascaridia, Oxyuris, Ancylostoma, Uncinaria, Toxascaris and Parascaris. Certain of these, such as Nematodirus, Cooperia, and Oesophagostomum attack primarily the intestinal tract while others, such as Haemonchus and Ostertagia, are more prevalent in the stomach while still others such as Dictyocaulus are found in the lungs. Still other parasites may be located in other tissues and organs of the body such as the heart and blood vessels, subcutaneous and lymphatic tissue and the like. The parasitic infections known as helminthiases lead to anemia, malnutrition, weakness, weight loss, severe damage to the walls of the intestinal tract and other tissues and organs and, if left untreated, may result in death of the infected host. The compounds of this invention have unexpectedly high activity against these parasites, and in addition are also active against Dirofilaria in dogs and cats, Nematospiroides, Syphacia, Aspiculuris in rodents, arthropod ectoparasites of animals and birds such as ticks, mites, lice, fleas, blowflies, in sheep Lucilia sp., biting insects and such migrating diperous larvae as Hypoderma sp. cattle, Gastrophilus in horses, and Cuterebra sp. in rodents and nuisance flies including blood feeding flies and filth flies.

The instant compounds are also useful against parasites which infect humans. The most common genera of parasites of the gastro-intestinal tract of man are Ancylostoma, Necator, Ascaris, Strongyloides, Trichinella, Capillaria, Trichuris, and Enterobius. Other medically important genera of parasites which are found in the blood or other tissues and organs outside the gastrointestinal tract are the filiarial worms such as Wuchereria, Brugia, Onchocerca and Loa, Dracunulus and extra intestinal stages of the intestinal worms Strongyloides and Trichinella. The compounds are also of value against arthropods parasitizing man, biting insects and other dipterous pests causing annoyance to man.

The compounds are also active against household pests such as the cockroach, Blatella sp., clothes moth, Tineola sp., carpet beetle, Attagenus sp., the housefly Musca domestica as well as fleas, house dust mites, termites and ants.

The compounds are also useful against insect pests of stored grains such as Tribolium sp., Tenebrio sp. and of agricultural plants such as aphids, (Acyrthiosiphon sp.); against migratory orthopterans such as locusts and immature stages of insects living on plant tissue. The compounds are useful as a nematocide for the control of soil nematodes and plant parasites such as Meloidogyne sp. which may be of importance in agriculture. The

compounds are also highly useful in treating acerage infested with fire ant nests. The compounds are scattered above the infested area in low levels in bait formulations which are broght back to the nest. In addition to a direct-but-slow onset toxic effect on the fire ants, the compound has a long-term effect on the nest by sterilizing the queen which effectively destroys the nest.

The compounds of this invention may be administered in formulations wherein the active compound is intimately admixed with one or more inert ingredients and optionally including one or more additional active ingredients. The compounds may be used in any composition known to those skilled in the art for administration to humans and animals, for application to plants and for premise and area application to control household pests in either a residential or commercial setting. For application to humans and animals to control internal and external parasites, oral formulations, in solid or liquid or parenteral liquid, implant or depot injection forms may be used. For topical application dip, spray, powder, dust, pour-on, spot-on, jetting fluid, shampoos, collar, tag or harness, may be used. For agricultural premise or area application, liquid spray, powders, dust, or bait forms may be used. In addition "feed-through" forms may be used to control nuisance flies that feed or breed in animal waste. The compounds are formulated, such as by encapsulation, to lease a residue of active agent in the animal waste which controls filth flies or other arthropod pests.

These compounds may be administered orally in a unit dosage form such as a capsule, bolus or tablet, or as a liquid drench where used as an anthelmintic in mammals. The drench is normally a solution, suspension or dispersion of the active ingredient usually in water together with a suspending agent such as bentonite and a wetting agent or like excipient. Generally, the drenches also contain an antifoaming agent. Drench formulations generally contain from about 0.001 to 0.5% by weight of the active compound. Preferred drench formulations may contain from 0.01 to 0.1% by weight. The capsules and boluses comprise the active ingredient admixed with a carrier vehicle such as starch, talc, magnesium stearate, or di-calcium phosphate.

Where it is desired to administer the instant compounds in a dry, solid unit dosage form, capsules, boluses or tablets containing the desired amount of active compound usually are employed. These dosage forms are prepared by intimately and uniformly mixing the active ingredient with suitable finely divided diluents, fillers, disintegrating agents, and/or binders such as starch, lactose, talc, magnesium stearate, vegetable gums and the like. Such unit dosage formulations may be varied widely with respect to their total weight and content of the antiparasitic agent depending upon factors such as the type of host animal to be treated, the severity and type of infection and the weight of the host.

When the active compound is to be administered via an animal feedstuff, it is intimately dispersed in the feed or used as a top dressing or in the form of pellets or liquid which may then be added to the finished feed or optionally fed separately. Alternatively, feed based individual dosage forms may be used such as a chewable treat. Alternatively, the antiparasitic compounds of this invention may be administered to animals parenterally, for example, by intraruminal, intramuscular, intravascular, intratracheal, or subcutaneous injection in which the active ingredient is dissolved or dispersed in a liquid carrier vehicle. For parenteral administration, the active material is suitably admixed with an acceptable vehicle, preferably of the vegetable oil variety such as peanut oil, cotton seed oil and the like. Other parenteral vehicles such as organic preparation using solketal, glycerol formal, propylene glycol, and aqueous parenteral formulations are also used. The active compound or compounds are dissolved or suspended in the parenteral formulation for administration; such formulations generally contain from 0.0005 to 5% by weight of the active compound.

Although the antiparasitic agents of this invention find their primary use in the treatment and/or prevention of helminthiasis, they are also useful in the prevention and treatment of diseases caused by other parasites, for example, arthropod parasites such as ticks, lice, fleas, mites and other biting arthropods in domesticated animals and poultry. They are also effective in treatment of parasitic diseases that occur in other animals including humans. The optimum amount to be employed for best results will, of course, depend upon the particular compound employed, the species of animal to be treated and the type and severity of parasitic infection or infestation. Generally good results are obtained with our novel compounds by the oral administration of from about 0.001 to 10 mg per kg of animal body weight, such total dose being given at one time or in divided doses over a relatively short period of time such as 1-5 days. With the preferred compounds of the invention, excellent control of such parasites is obtained in animals by administering from about 0.025 to 0.5 mg per kg of body weight in a single dose. Repeat treatments are given as required to combat re-infections and are dependent upon the species of parasite and the husbandry techniques being employed. The techniques for administering these materials to animals are known to those skilled in the veterinary field.

When the compounds described herein are administered as a component of the feed of the animals, or dissolved or suspended in the drinking water, compositions are provided in which the active compound or compounds are intimately dispersed in an inert carrier or diluent. By inert carrier is meant one that will not react with the antiparasitic agent and one that may be administered safely to animals. Preferably, a carrier for feed administration is one that is, or may be, an ingredient of the animal ration.

Suitable compositions include feed premixes or supplements in which the active ingredient is present in relatively large amounts and which are suitable for direct feeding to the animal or for addition to the feed either directly or after an intermediate dilution or blending step. Typical carriers or diluents suitable for such compositions include, for example, distillers' dried grains, corn meal, citrus meal, fermentation residues, ground oyster shells, wheat shorts, molasses solubles, corn cob meal, edible bean mill feed, soya grits, crushed limestone and the like. The active compounds are intimately dispersed throughout the carrier by methods such as grinding, stirring, milling or tumbling. Compositions containing from about 0.005 to 2.0% weight of the active compound are particularly suitable as feed premixes. Feed supplements, which are fed directly to the animal, contain from about 0.0002 to 0.3% by weight of the active compounds.

Such supplements are added to the animal feed in an amount to give the finished feed the concentration of active compound desired for the treatment and control of parasitic diseases. Although the desired concentration of active compound will vary depending upon the factors previously mentioned as well as upon the particular compound employed, the compounds of this invention are usually fed at concentrations of between 0.00001 to 0.002% in the feed in order to achieve the desired anti-parasitic result.

In using the compounds of this invention, the individual compounds may be prepared and used in that form. Alternatively, mixtures of the individual compounds may be used, or other active compounds not related to the compounds of this invention.

The compounds of this invention are also useful in combatting agricultural pests that inflict damage upon crops while they are growing or while in storage. The compounds are applied using known techniques as sprays, dusts, emulsions and the like, to the growing or stored crops to effect protection from such agricultural pests.

The following examples are provided in order that this invention might be more fully understood; they are not to be construed as limitative of the invention.

## General

Analytical thin layer chromatography (TLC) was performed on EM Reagents 0.25 mm silica gel 60-F plates. Visualization was accomplished with UV light and by dipping in an aqueous ceric ammonium molybdate solution followed by heating. Solvents for extraction were reagent grade. Solvents for reactions were dried with 3-Å or 4-Å molecular sieves. All reactions were performed under an inert atmosphere of dry nitrogen in dry glassware. $^1$H-NMR spectra were recorded in deuterochloroform on a Varian XL-300 (299.94 MHz) spectrometer. Chemical shifts are reported in ppm from an internal standard of residual chloroform (7.27 ppm). Selected data are reported as follows: chemical shift, multiplicity (s = singlet, d = doublet, t = triplet, q = quartet, m = multiplet, br = broadened, obs = obscured), coupling constants (Hz), and assignments. Assignments were made with the aid of 2D (COSY) data. $^{13}$C NMR spectra were recorded in deuterochlorofrom on a Varian XL-300 (75.4 MHz) spectrometer. Chemical shifts are reported in ppm from the central peak of deuterochlorofrom (77.0 ppm). Assignments were made with the aid of APT data. Data are reported as follows: chemical shift, assignment. Combustion analyses were obtained from Microlit Laboratories, Inc., Caldwell, New Jersey.

## EXAMPLE 1

### 5-O-tert-Butyldimethylsilyl-13-O-toluenesulfonyl-22,23-dihydroavermectin B1 aglycone (2)

A 25 mL flask fitted with a nitrogen inlet tube and Teflon coated stirring bar was charged with 5-O-tert-butyldimethylsilyl-22,23-dihydroavermectin B1 aglycone, 1 (1.28 g, 1.82 mmol), p-toluenesulfonic anhydride (1.19 g, 3.65 mmol), N,N-diisopropylethyl amine (0.95 mL, 0.70 g, 5.5 mmol), N,N-dimethylaminopyridine (445 mg, 3.65 mmol) and dichloromethane (5.1 mL). The resulting solution was stirred at 20°C for 24 hours. (The tosylation can be monitored by removing a 20 μL aliquot, dissolving it in 0.6 mL of deuterochloroform, obtaining a $^1$H-NMR and integrating the signal for $H_{13}$; $H_{13}$ of 1: 3.98 (br s); $H_{13}$ of 2: 4.82 (br s)). The resulting mixture was cooled to 0°C and hexane (10.2 mL) was added followed by aqueous sodium hydrogen sulfate (10 mL, 1 N) at a rate to maintain the internal temperature at or below 5°C (~10 min). The resulting pH was about 2. The layers were separated and the aqueous layer was extracted with 2:1 hexane:dichloromethane (15 mL). The organic layers were individually washed with 15 mL of saturated aqueous sodium bicarbonate and 15 mL of water. The organic layers were combined, dried over sodium sulfate, filtered and concentrated to provide 2 (about 2 g, impure) as an orange solid. This material was used as is in subsequent reactions after concentrating it from dry toluene (2 x 10 mL).

EXAMPLE 2

5-O-tertButyldimethylsilyl-13β-nitrooxy-22,23-dihydroavermectin B1 aglycone (3)

A 25-mL flask fitted with a nitrogen inlet tube and teflon coated stirring bar was charged with 5-O-tert-butyldimethylsilyl-13-O-p-toluenesulfonyl-22,23-dihydroavermectin B1 aglycone, 2 (~2 g, see above, ~1.82 mmol, crude mixture from previous experimental procedure), tetra-n-butylammonium nitrate (1.66 g, 5.45 mmol) and toluene (8 mL). The resulting solution was stirred at 40°C for 96 hours. (The displacement can be monitored by TLC: $R_f$, starting material = 0.40; $R_f$, nitrate ester = 0.57 in 4:1 hexane:ethyl acetate). The resulting mixture was cooled to 20°C, concentrated to ~3 mL and chromatographed twice (5 x 30 cm column; 92:8 hexane: ethyl acetate) to provide 3 (774 mg, 57%). The impurities have slightly higher $R_f$'s than the desired nitrate ester ($\Delta R_f$ ~ 0.07 in 92:8 hexane:ethyl acetate. NMR analysis of the isolated three component impurity mixture (~5%) was complicated since this mixture was not readily separable. Data for 3:

$^1$H-NMR: δ 5.85 (dd, J=14.3, 11.4, $H_{10}$), 5.60 (dt, J=11.3, 1.9, $H_9$), 5.42 (m, $H_{15}$), 5.30 (m, $H_3$, $H_{11}$), 5.20 (m, $H_{19}$), 4.95 (d, J=10.6, $H_{13}$), 4.65 (dd, J=14.4, 1.9, $H_{8a}$), 4.54 (dd, J=14.4, 1.9, $H_{8a}$), 4.40 (m, H5), 4.00 (s, OH), 3.79 (d, J=5.5, $H_6$), 3.60 (m, $H_{17}$), 3.32 (m, $H_2$), 3.14 (m, $H_{25}$), 2.55 (m, $H_{12}$), 2.30 (m, 2 x $H_{16}$), 1.99 (dd, J=11.8, 3.6, $H_{20eq}$), 1.77 (br s, 3 x $H_{4a}$), 1.55 (s, 3 x $H_{14a}$), 1.10 (d, J=6.5, $H_{12a}$), 0.94 (t, J=7.3, 3 x $H_{28}$), 0.90 (s, $SiC(CH_3)_3$), 0.82 (d, J=6.6, $H_{26a}$), 0.10 (s, $Si(CH_3)_2$). $^{13}$C-NMR: $\delta_c$ 173.6 ($C_1$), 142.5, 137.5, 133.3 ($C_4$, $C_8$, $C_{14}$), 135.4 ($C_{11}$), 129.0, 125.5, 118.7, 117.3 ($C_3$, $C_9$, $C_{10}$, $C_{15}$), 97.5 ($C_{21}$), 93.1 ($C_{13}$), 80.2 ($C_6$), 80.1 ($C_7$), 77.4 ($C_{25}$), 69.3, 68.6, 66.6 ($C_5$, $C_{17}$, $C_{19}$), 67.7 ($C_{8a}$), 45.6 ($C_2$), 42.3 ($C_{20}$), 36.6, 35.7, 34.7 ($C_{18}$, $C_{22}$, $C_{26}$), 35.7 ($C_{16}$), 31.2 ($C_{24}$), 28.0 27.5 ($C_{23}$, $C_{27}$), 25.6 ($SiC(CH_3)_3$), 20.1, 18.8, 17.5 ($C_{4a}$, $C_{12a}$, $C_{24a}$), 12.6, 11.7, 11.0 ($C_{14a}$, $C_{26a}$, $C_{28}$). MS (EI, 70 eV): 745 (M+, 5), 503 (18), 307 (30), 225 (30), 223 (32), 195 (70), 151 (72), 137 (72), 95 (100), 75 (70), 73 (80), 69 (66), 55 (66).

Anal. Calcd. for $C_{40}H_{63}NO_{10}Si$:
C, 64.40; H, 8.51; N, 1.88.
Found: C, 64.14; H, 8.58; N, 1.79.

EXAMPLE 3

5-O-tert-Butyldimethylsilyl-13-epi-22,23-dihydro-avermectin B1 aglycone (4)

Zinc powder (~1 g) was stirred for 5 minutes in 1 N HCl, filtered and washed with water (3 x 10 mL; final pH > 6), methanol (2 x 10 mL) and toluene (2 x 5 mL). The resulting material was air dried and a portion of it was used in the following reduction. A 15 mL flask fitted with a nitrogen inlet tube and teflon coated stirring bar was charged with 5-O-tert-butyldimethylsilyl-13β-nitrooxy-22,23-dihydroavermectin B1 aglycone, 3 (513 mg, 0.688 mmol), tetrahydrofuran (4.3 mL) and acetic acid (0.7 mL). The solution was stirred in a water bath at 20°C and zinc (450 mg, 6.88 mmol, see above) was added in one portion. The resulting suspension was stirred for 10 minutes as the temperature rose to 25°C. (The reduction can be monitored by TLC: $R_f$, nitrate ester = 0.50; $R_f$, β-alcohol = 0.29; $R_f$, α-alcohol = 0.19 in 4:1 hexane:ethyl acetate). The resulting mixture was filtered through a coarse glass frit and the solids were washed with 3 x 7 mL of ethyl acetate. The washings were combined and cautiously washed with saturated aqueous sodium bicarbonate (2 x 15 mL). The organic layer was dried over sodium sulfate, filtered, concentrated and chromatographed (3 x 30 cm column; 80:20 hexane:ethyl acetate) to provide 4 (425 mg, 88%). The sample of 4 prepared in this manner was identical (TLC, $^1$H NMR) to that previously reported for an authentic sample.

$^1$H-NMR: δ 5.75 (m, $H_9$, $H_{10}$), 5.30 (m, $H_3$, $H_{11}$), 5.20 (m, $H_{15}$, $H_{19}$), 4.65 (dd, J=14.4, 1.9, $H_{8a}$), 4.54 (dd, J=14.4, 1.9, $H_{8a}$), 4.42 (m, $H_5$), 3.98 (OH), 3.78 (d, J=5.5, $H_6$), 3.70 (d, J=10.0, $H_{13}$), 3.55 (m, $H_{17}$), 3.35 (m, $H_2$), 3.15 (m, $H_{25}$), 2.35 (m, $H_{12}$), 1.78 (s, 3 x $H_{4a}$), 1.55 (s, 3 x $H_{14a}$), 1.12 (d, J=6.5, 3 x $H_{12a}$), 0.94 (t, J=7.3, 3 x $H_{28}$), 0.90 (S, $SiC(CH_3)_3$), 0.82 (d, J=6.6, $H_{26a}$), 0.10 (s, $Si(CH_3)_2$). $^{13}$C-NMR: $\delta_c$ 173.8 ($C_1$), 140.9, 140.2, 137.4 ($C_4$, $C_8$, $C_{14}$), 138.6 ($C_{11}$), 123.9, 123.3 ($C_9$, $C_{10}$), 119.3, 117.4 ($C_3$, $C_{15}$), 97.5 ($C_{21}$), 83.4 ($C_{13}$), 80.3 ($C_6$),80.1 ($C_7$), 77.1 ($C_{25}$), 69.4, 68.7, 67.1 ($C_5$, $C_{17}$, $C_{19}$), 68.0 ($C_{8a}$), 45.7 ($C_2$), 41.6 ($C_{12}$), 41.4 ($C_{20}$), 36.5, 35.7, 34.5 ($C_{16}$, $C_{18}$, $C_{22}$), 35.5 ($C_{26}$), 28.1, 27.4 ($C_{23}$, $C_{27}$), 25.9 ($SiC(CH_3)_3$), 20.1, 19.0, 17.5 ($C_{4a}$, $C_{12a}$, $C_{24a}$), 18.4 ($SiC(CH_3)_3$), 12.5, 11.9, 10.6 ($C_{14}$, $C_{26a}$, $C_{28}$).

Anal. Calcd for $C_{40}H_{64}O_8Si$:
C, 68.53; H, 9.20.
Found: C, 68.15; H, 9.28.

EXAMPLE 4

13β-Nitrooxy-22,23-dihydroavermectin B1 aglycone.

A 25-mL polypropylene vial fitted with a magnetic stirring bar was charged with 5-0-tert-butyl-dimethylsilyl-13β-nitrooxy-22,23-dihydroavermectin B1 aglycone (103 mg, 0.138 mmol), hydrogen fluoridepyridine (1 mL of a mixture of 27.5 mL tetrahydrofuran, 12.5 mL of pyridine and 25 g of commercially available hydrogen fluoride-pyridine complex) and tetrahydrofuran (3 mL). The reaction was stirred at room temperature for 17 hours, then quenched by the cautious addition of saturated aqueous potassium carbonate (10 mL). The resulting mixture was extracted with dichloromethane (3 x 10 mL). The organic layers were individually washed with aqueous sodium hydrogen sulfate (1.0 M, 10 mL) and saturated aqueous sodium chloride (10 mL). The organic layers were combined, dried over sodium sulfate, filtered, concentrated and chromatographed (2 x 30 cm column; 67:33 hexane:ethyl acetate) to provide 13β-nitrooxy-22,23-dihydroavermectin B1 aglycone (57 mg, 66%).
$^1$H NMR: δ 5.85 (m, $H_{10}$, $H_9$), 5.50-5.20 (m, $H_3$, $H_{11}$, $H_{15}$, $H_{19}$), 4.95 (d, J=10.8, $H_{13}$), 4.68 (br S, $H_{8a}$), 4.27 (m, $H_5$), 3.99 (s, OH), 3.95 (d, J=6.3, $H_6$), 3.60 (m, $H_{17}$), 3.25 (m, $H_2$), 3.14 (m, $H_{25}$), 2.55 (m, $H_{12}$), 2.30 (m, 2 x $H_{16}$), 1.99 (dd, J=11.8, 3.6, $H_{20eq}$), 1.77 (br s, 3 x $H_{4a}$), 1.55 (s, 3 x $H_{14a}$), 1.10 (d, J=6.5 $H_{12a}$), 0.94 (t, J=7.3, 3 x $H_{28}$), 0.82 (d, J=6.6, $H_{26a}$).
$^{13}$C NMR: $\delta_c$ 173.4 ($C_1$), 141.9, 137.9, 133.3 ($C_4$, $C_8$, $C_{14}$), 135.9 ($C_{11}$), 129.0, 125.5, 119.7, 118.0 ($C_3$, $C_9$, $C_{10}$, $C_{15}$), 97.5 ($C_{21}$), 93.1 ($C_{13}$), 80.3 ($C_6$), 79.3 ($C_7$), 77.5 ($C_{25}$), 68.5, 67.6, 66.6 ($C_5$, $C_{17}$, $C_{19}$), 68.4 ($C_{8a}$), 45.6 ($C_2$), 41.3 ($C_{20}$), 38.3 ($C_{12}$), 36.7, 35.7 31.2 ($C_{18}$, $C_{22}$, $C_{26}$), 35.5 ($C_{16}$), 31.2 ($C_{24}$), 28.0, 27.5 ($C_{23}$, $C_{27}$), 20.0, 18.8, 17.5 ($C_{4a}$, $C_{12a}$, $C_{24a}$), 12.6, 11.7, 10.9 ($C_{14a}$, $C_{26a}$, $C_{28}$).
MS (EI, 70 eV): 631 (M+, 5), 503 (13), 307 (25), 223 (29), 195 (33), 179 (37), 151 (95) 137 (66), 95 (100), 69 (45), 50 (58).

Anal Calcd for $C_{34}H_{49}NO_{10}$:
C, 64.64; H, 7.82; N, 2.22.
Found: C, 64.45; H, 7.60; N, 2.01.

EXAMPLE 5

13-Epi-22,23-dihydroavermectin B1 aglycone.

A solution of 5-0-tert-butyldimethylsilyl-13-epi-22,23-dihydroavermectin B1 aglycone (710 mg) in methanol (20 mL) containing 1% of p-toluenesulfonic acid monohydrate (200 mg) was stirred for 17 hours at room temperature. Saturated aqueous sodium bicarbonate was added and the resulting mixture was extracted with ethyl acetate. The organic layer was washed with water, dried over sodium sulfate, concentrated and chromatographed to provide 13-epi-22,23-dihydroavermectin B1 aglycone.

EXAMPLE 6

5,23-Bis[O-tert-butyldimethylsilyl]avermectin B2 aglycone.

Avermectin B2 aglycone (252 mg, 0.418 mmol) was dissolved in dry N,N-dimethylformamide (4.5 mL) and imidazole (338 mg, 4.96 mmol) and tert-butyldimethylsilyl chloride (316 mg, 2.10 mmol) were added sequentially. The resulting mixture was warmed to 35°C and stirred for 5 hours. The reaction mixture was cooled to room temperature and water (50 mL) was added. The resulting mixture was extracted with diethyl ether (3 x 30 mL). The organic layers were combined, dried over sodium sulfate, filtered and chromatographed (3 x 20 cm column, 3:1 hexanes: diethyl ether) to provide 5,23-Bis[0-tert-butyldimethylsilyl]avermectin B2 aglycone (108 mg, 31%).

EXAMPLE 7

5-O-Tert-butyldimethylsilylavermectin B1 aglycone.

If avermectin B1 aglycone is reacted according to the procedure of Example 6, one obtains 5-O-tert-butyldimethylsilylavermectin B1 aglycone, which is characterized by its mass and NMR spectra.

EXAMPLE 8

13β-Nitrooxyavermectin B1 aglycone.

If 5-O-tert-butyldimethylsilylavermectin B1 aglycone is reacted according to the procedures fully described in Examples 1, 2, and 4, one obtains 13-beta-nitrooxyavermectin B1 aglycone, which is characterized by its mass and NMR specta.

EXAMPLE 9

13-epi-Avermectin B1 aglycone.

If 5-O-tert-butyldimethylsilylavermectin B1 aglycone is reacted according to the procedures fully described in Examples 1, 2, 3, and 5, one obtains 13-epi-avermectin B1 aglycone.

EXAMPLE 10

13β-Nitrooxy-5-oxoavermectin B1 aglycone.

A solution of 100 mg of 13β-nitrooxy avermectin B1 aglycone (from Example 8) in 3.5 ml of anhydrous dimethylformamide is stirred with 82 mg of pyridinium dichromate at room temperature for 45 minutes. The reaction is worked up with water and ether, and the washed ether phase is concentrated in vacuo to a colorless glass, which is characterized by its mass and NMR spectra as 13-beta-nitrooxy-5-oxoavermectin B1 aglycone.

EXAMPLE 11

13β-Nitrooxyavermectin B1 aglycone 5-ketoxime.

A solution containing 130 mg of 13-beta-nitrooxy-5-oxoavermectin B1 aglycone in 5.0 ml of absolute ethanol, 0.5 ml of pyridine, and 104 mg of hydroxylamine hydrochloride is stirred at room temperature for 3.5 hours. Then the reaction mixture is concentrated in vacuo at room temperature to a thick oil. This is dissolved in ether, and the solution is washed with water, dried over $MgSO_4$, and concentrated in vacuo to a light foam. Purification by preparative silica gel layer chromatography with a methylene chloride-methanol solvent mixture gives 13β-nitrooxyavermectin B1 aglycone 5-ketoxime, which is characterized by its mass and 1H-NMR specta.

## Claims

1. A process for the inversion of the natural α- configuration of the 13-position hydroxy groups of avermectin aglycone compounds which comprises, in Step A, treating a compound having the formula:

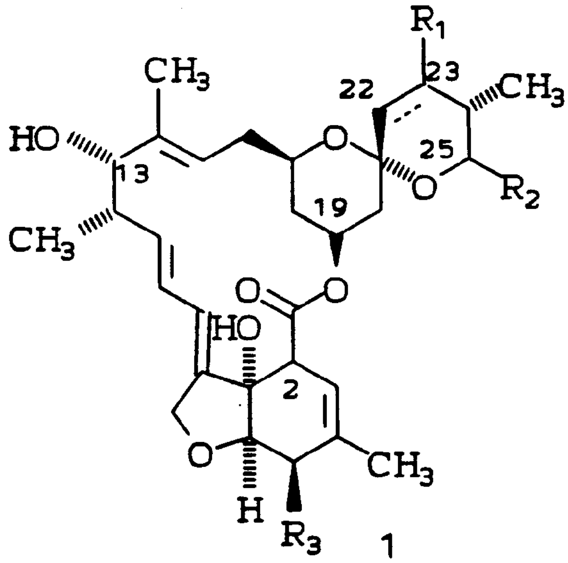

1

where the broken line at the 22,23-position indicates either a single or a double bond at the 22,23-position; $R_1$ is present only when the broken line indicates a 22,23-single bond and is hydrogen, hydroxy or oxo; $R_2$ is loweralkyl, loweralkenyl or cycloloweralkyl; and $R_3$ is hydroxy, loweralkoxy, oximino or oxo; with an acylating reagent to form a compound having the formula:

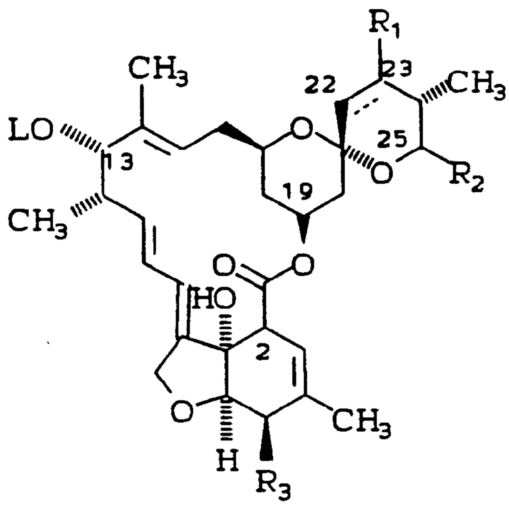

where L is a leaving group, which is then treated, in Step B, with a tetraloweralkyl Ammonium nitrate to form a 13-β nitrooxy derivative of the formula:

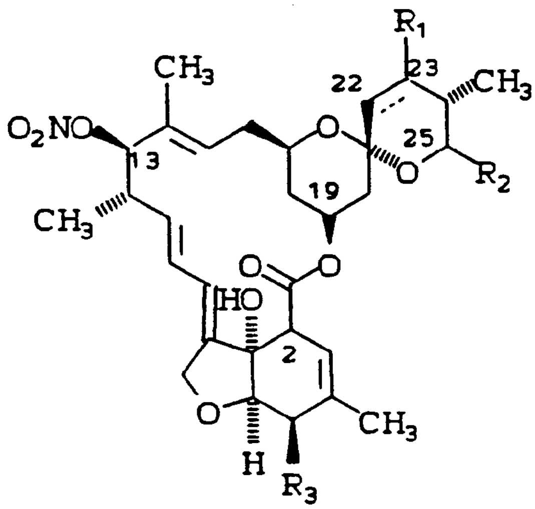

which is reduced, in Step C, with zinc in the presence of an acid to form the 13-β-hydroxy derivative of the formula:

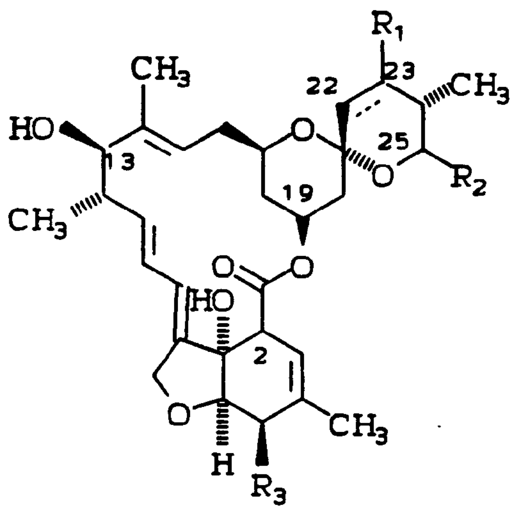

where $R_1$, $R_2$ and $R_3$ are as defined above.

2. The process of Claim 1 where the leaving group for Step A is a sulfonic acid derivative, the tetraloweralkyl ammonium nitrate in Step B is tetrabutyl ammonium nitrate and the acidic medium in Step C is acetic acid.

3. A compound having the formula:

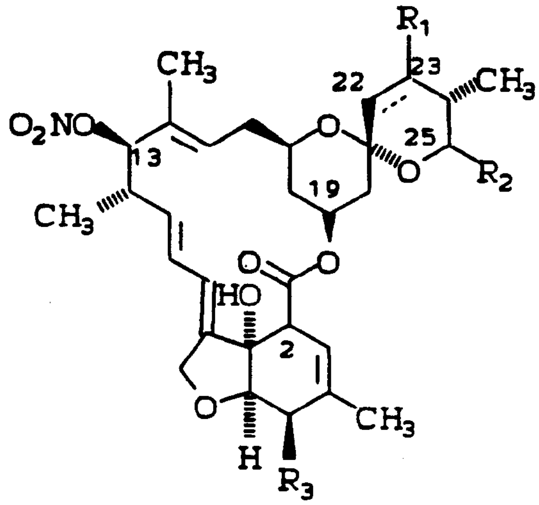

where the broken line at the 22,23-position indicates either a single or a double bond at the 22,23-position;

$R_1$ is present only when the broken line indicates a 22,23-single bond and is hydrogen, hydroxy or oxo;

$R_2$ is loweralkyl, loweralkenyl or cycloloweralkyl; and

$R_3$ is hydroxy, loweralkoxy, oximino or oxo.

4. A compound of Claim 3 where the broken line indicates a 22,23-single bond and $R_1$ is hydrogen, hydroxy or oxo;
$R_2$ is alkyl of from 3 to 5 carbon atoms or cycloalkyl of from 4 to 6 carbon atoms; and
$R_3$ is hydroxy, methoxy, oximino or oxo.

5. A compound of Claim 4 where $R_1$ is hydrogen or oxo;
$R_2$ is alkyl of 3 to 4 carbon atoms or cycloalkyl of 5 to 6 carbon atoms; and
$R_3$ is hydroxy or oximino.

6. A compound of Claim 5 where $R_1$ is hydrogen;
$R_2$ is iso-propyl, sec-butyl, cyclohexyl or cyclopentyl; and
$R_3$ is hydroxy.

7. A process for the preparation of a compound of Claim 3 which comprises treating a compound having the formula:

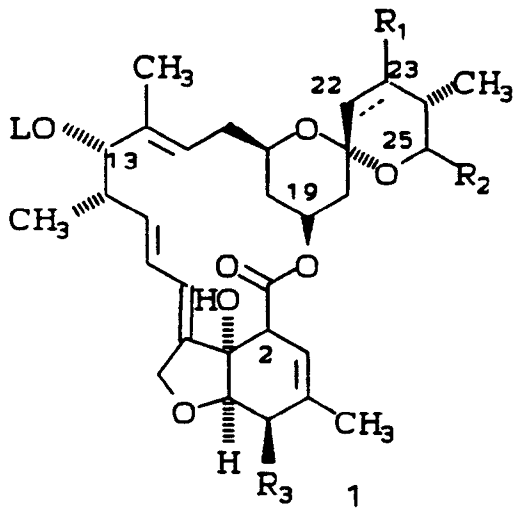

1

where L is a leaving group and $R_1$, $R_2$ and $R_3$ are as defined in Claim 3.

**8.** The use of a compound of Claim 3 for the manufacture of a medicament for the treatment of parasitic infections of humans and animals.

**9.** A method for the treatment of parasitic infestations of plants or plant crops which comprises treating said plants or plant crops or the soil in which said plants grow, with an effective amount of a compound of Claim 3.

**10.** A composition useful for the treatment of parasitic infections of human or animals or for the treatment of parasitic infestations of plants or plant crops which comprises an inert carrier and an effective amount of a compound of Claim 3.

European Patent Office

# EUROPEAN SEARCH REPORT

Application Number

EP 92 30 4151

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| D,A | JOURNAL OF MEDICINAL CHEMISTRY. vol. 32, no. 2, February 1989, WASHINGTON US pages 375 - 381; H. MROZIK ET AL.: 'Syntheses and biological activities of 13-substituted avermectin aglycons.' * abstract ; page 376, right column, lines 9-37 , chart I, compounds 1,4,7 * | 1,3,8 | C07H19/01<br>C07D493/22<br>A01N43/90<br>A61K31/70<br>//(C07D493/22, 313:00,311:00, 311:00,307:00) |
| D,A | TETRAHEDRON LETTERS. vol. 26, no. 28, 1985, OXFORD GB pages 3369 - 3372; G. CAINELLI ET AL.: 'Inversion of configuration of alcohols through nucleophilic displacement promoted by nitrate ions' * abstract * | 1,2 | |
| P,X | EP-A-0 428 285 (MERCK & CO.) * claim 6; page 7, lines 30-42 * | 1 | |
| | | | TECHNICAL FIELDS SEARCHED (Int. Cl.5 ) |
| | | | C07H<br>C07D<br>A61K<br>A01N |

The present search report has been drawn up for all claims

| Place of search | Date of completion of the search | Examiner |
|---|---|---|
| THE HAGUE | 09 JULY 1992 | VOYIAZOGLOU D. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)